# EUROPÄISCHE PATENTSCHRIFT

(11) **EP 1 373 478 B1**
(45) Veröffentlichungstag und Bekanntmachung des Hinweises auf die Patenterteilung: **19.04.2006**
(21) Anmeldenummer: 02710669.9
(22) Anmeldetag: 11.02.2002
(51) Int. Cl.: C12N 7/04, A61K 39/00, C07K 14/18, C12N 15/40

(54) **ATTENUIERTE LEBENDIMPFSTOFFE**
ATTENUATED LIVE VACCINE
VACCINS ATTENUES A VIRUS VIVANTS

(30) Priorität: 21.02.2001 AT 2722001
(43) Veröffentlichungstag der Anmeldung: 02.01.2004
(73) Patentinhaber: Intercell AG, 1030 Wien (AT)
(72) Erfinder: Heinz, Franz X., 1090 Wien (AT); Mandl, Christian, 1090 Wien (AT)
(74) Vertreter: Sonn & Partner Patentanwälte
(86) Internationale Anmeldenummer: PCT/AT2002/000046
(87) Internationale Veröffentlichungsnummer: WO 2002/066621

(56) Entgegenhaltungen:
- EP-A- 1 018 556
- US-B1- 6 184 024
- MARKOFF LEWIS ET AL: "A conserved internal hydrophobic domain mediates the stable membrane integration of the dengue virus capsid protein." VIROLOGY, Bd. 233, Nr. 1, 1997, Seiten 105-117, XP002201374 ISSN: 0042-6822
- HOPE R GRAHAM ET AL: "Sequence motifs required for lipid droplet association and protein stability are unique to the hepatitis C virus core protein." JOURNAL OF GENERAL VIROLOGY, Bd. 81, Nr. 8, August 2000 (2000-08), Seiten 1913-1925, XP001077721 ISSN: 0022-1317
- KOFLER REGINA M ET AL: "Capsid protein C of tick-borne encephalitis virus tolerates large internal deletions and is a favorable target for attenuation of virulence." JOURNAL OF VIROLOGY. UNITED STATES APR 2002, Bd. 76, Nr. 7, April 2002 (2002-04), Seiten 3534-3543, XP001073714 ISSN: 0022-538X
- MANDL C.W. ET AL: 'Spontaneous and engineered deletions in the 3' noncoding region of tick-borne encephalitis virus: Construction of highly attenuated mutants of a flavivirus' JOURNAL OF VIROLOGY Bd. 72, Nr. 3, M{rz 1998, Seiten 2132 - 2140, XP002151482

## Beschreibung

Die Erfindung betrifft attenuierte Flaviviren-Lebendimpfstoffe.

Die Familie Flaviviridae umfasst drei Genera, das Genus Flaviviren, das Genus Pestiviren und das Genus Hepaciviren.

Dem Genus Flaviviren gehören in der Mehrzahl durch Stechmücken oder Zecken übertragene Viren an, von denen viele bedeutende Krankheitserreger des Menschen, aber auch von Tieren sind. Von besonderer Bedeutung sind das Gelbfieber (GF)-Virus, das Virus der Japanischen Enzephalitis (JE), die vier Serotypen der Dengue (Den)-Viren, das Frühsommermeningoenzephalitis (FSME, engl. tick-borne encephalitis, TBE) Virus, und auch das West Nil (WN) Virus, das in jüngster Zeit auch in Nordamerika als Krankheitserreger bei Mensch und verschiedenen Vogelarten in Erscheinung getreten ist.

Das Genus der Pestiviren enthält Tierpathogene von großer wirtschaftlicher Bedeutung, nämlich das klassische Schweinefieber (CSF) Virus, das bovine virale Diarrhoe (BVD)-Virus und das border disease Virus (BDV).

Das Genus Hepaciviren umfasst die verschiedenen Subtypen des Hepatitis C Virus (HCV) und verwandte Viren wie das Hepatitis G Virus (HGV).

Diese drei Genera werden zur Familie der Flaviviridae zusammengefasst, da alle Vertreter dieser Familie einen nahezu identen Genomaufbau aufweisen und in zahlreichen strukturellen und funktionellen Eigenschaften übereinstimmen. Alle Flaviviren sind relativ kleine, umhüllte Viren, die ein einzelsträngiges RNA Molekül mit mRNA-Polarität als Genom aufweisen. Das Genom besitzt einen langen offenen Leserahmen, der für alle Proteine in Form eines Polyproteins kodiert. Die einzelnen reifen Virusproteine werden durch die Aktivität viraler und zellulärer Proteasen gebildet. Die Anordnung der einzelnen Virusproteine im Genom ist für alle Flaviviren gleich und beginnt am 5'-Ende mit dem Kapsidprotein, den Oberflächenproteinen und einer Reihe von Nichtstrukturproteinen, deren letztes die virale Polymerase ist. Als Besonderheit enthalten die Pestiviren noch eine Autoprotease vor dem Kapsidprotein. Das Nukleokapsid der Flaviviren wird nur durch ein einziges virales Protein, nämlich das Kapsidprotein gebildet und umschließt das virale Genom. Es wird angenommen, dass das Kapsid eine ikosaedrische Symmetrie aufweist.

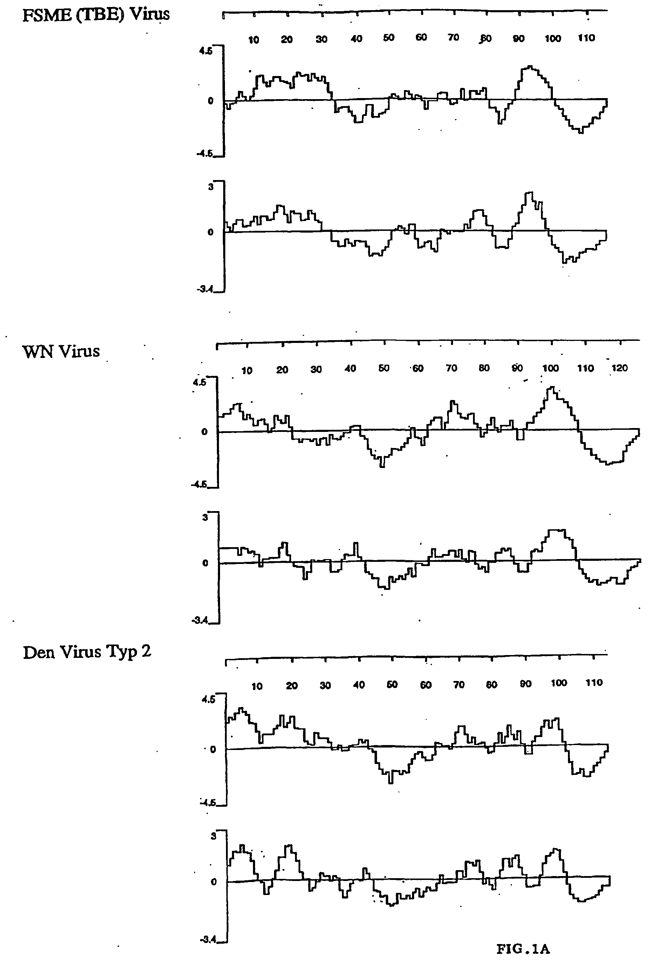

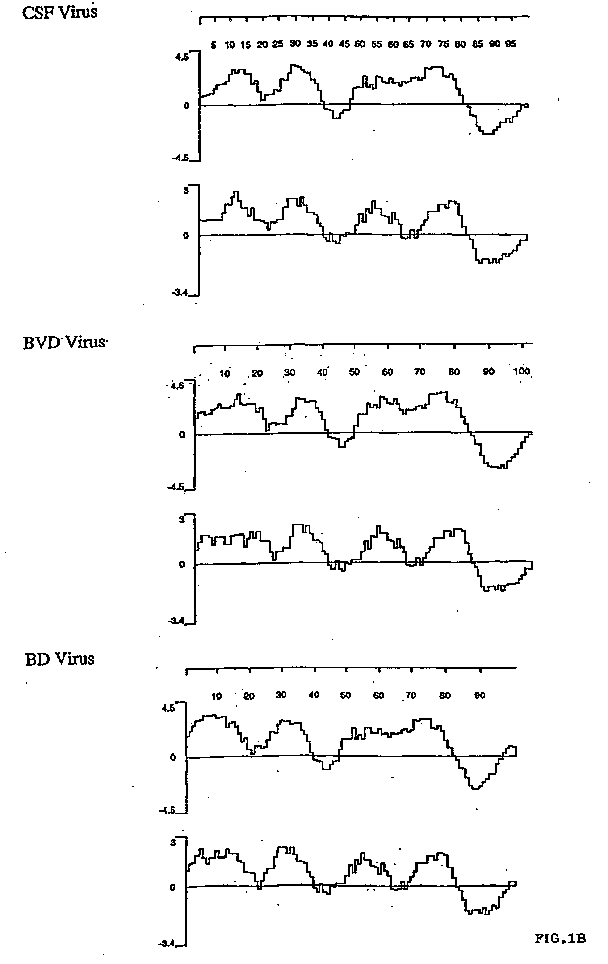

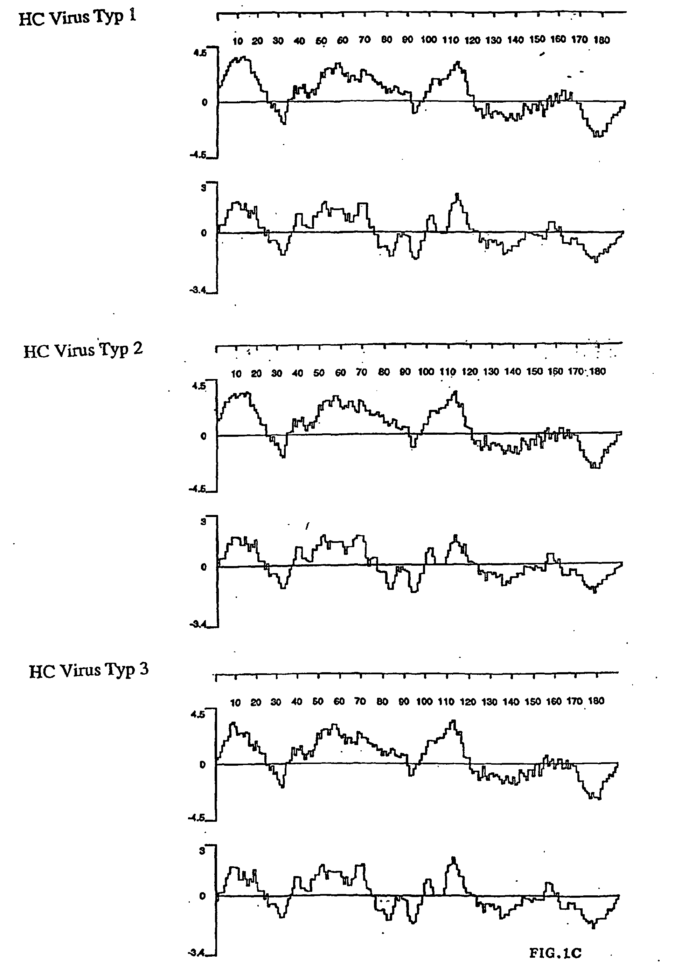

Die genaue dreidimensionale Struktur des Kapsidproteins ist bis jetzt für keines der Flaviviren bekannt. Die bekannten Aminosäuresequenzen weisen aber zahlreiche Übereinstimmungen auf, sodass es sehr wahrscheinlich ist, dass die Kapsidproteine zahlreiche strukturelle Ähnlichkeiten aufweisen. Hierbei sind die Ähnlichkeiten bei Vertretern des selben Genus naturgemäß noch größer als zwischen Vertretern verschiedener Genera. Das Kapsidprotein ist in allen Fällen ein eher kleines Protein mit einer Länge von ungefähr 100 bis 190 Aminosäuren. Es weist einen ungewöhnlich hohen Anteil basischer Aminosäuren, also der Aminosäuren Lysin und Arginin, auf. Es wird angenommen, dass die basischen Aminosäuren für die Interaktion mit der viralen RNA wichtig sind (Khromykh und Westaway, 1996). Alle Flavivirus-Kapsidproteine besitzen aber auch charakteristische hydrophobe Abschnitte (Fig. 1). Ein solcher hydrophober Abschnitt wird stets von den carboxy-terminalen ungefähr 20 Aminosäuren gebildet. Dieser Abschnitt dient als interne Signalsequenz für das in der Genomsequenz folgende Oberflächen-Strukturprotein. Durch diese Signalsequenz, die während der Proteinsynthese in die Membran des endoplasmatischen Retikulums integriert wird, wird das Kapsidprotein zunächst in der Membran verankert. Der Anker wird später proteolytisch abgespalten. Zusätzlich gibt es interne hydrophobe Abschnitte. Bei Vertretern des Genus Flaviviren wurde die funktionelle Bedeutung einer internen hydrophoben Domäne beschrieben (Markoff et al., 1997). Die Grenzen dieser Domäne werden von den Autoren für eine Reihe von Flaviviren wie folgt angegeben: Dengue 1: 46-67, Dengue 2: 46-66, Dengue 3: 46-67, Dengue 4: 45-65, Japanese Encephalitis: 46-62, West Nil: 46-62, Murray Valley Encephalitis: 46-62, Saint Louis Encephalitis: 45-61, Gelbfieber: 43-58, Langat: 42-54, Powassan: 40-52, FSME: 42-54. Auch für Hepatitis C Virus wurde eine funktionell wichtige interne hydrophobe Domäne identifiziert (Hope and McLauchlan, 2000), die von Aminosäure 119 bis 145, speziell von 125 bis 144, reicht. Auch Pestiviren besitzen kurze interne Abschnitte mit vornehmlich hydrophobem Charakter.

Gegen einige Flaviviren werden Impfstoffe erfolgreich eingesetzt. So gibt es z.B. gegen das GF Virus, das JE Virus und das CSF Virus Lebendimpfstoffe, gegen JE und FSME werden Totimpfstoffe eingesetzt. Angesichts der großen Bedeutung der Flaviviren in der Human- und Veterinärmedizin besteht ein großer Bedarf an der Entwicklung neuer und verbesserter Vakzinen.

Es sind eine Reihe von attenuierten Flaviviren bekannt, deren Attenuierung auf Mutationen in verschiedenen Bereichen des Genoms beruht. Attenuierende Mutationen wurden entweder in natürlich vorkommenden Stämmen beobachtet, durch Serienpassagen von Viren im Labor erhalten, durch Selektion von Mutanten in Gegenwart neutralisierender Antikörper, oder durch gezielte Einführung von Mutationen mit Hilfe rekombinanter Klonierungstechniken hergestellt. Von etlichen Flaviviren sind infektiöse cDNA Klone vorhanden und es ist dem Fachmann bekannt wie solche Klone herzustellen sind. Mit Hilfe dieser infektiösen cDNA Klone können nach dem Stand der Technik Mutationen in das Genom von Flaviviren spezifisch eingeführt werden.

Bekannte Mutationen zur Attenuierung von Flaviviren finden sich in folgenden Genomabschnitten:

Hüllproteine: Die meisten Beobachtungen attenuierender Mutationen betreffen das Hüllprotein E (Genus Flavivirus) (zusammengefasst in McMinn, 1997; neu z.B. Mandl et al., 2000). Ebenso wurden attenuierende Mutationen in Protein E(ms) (Genus Pestivirus) beschreiben (Meyers et al., 1999).

Nichtstrukturproteine: Eine Punktmutation in Protein NS 1 des Kunjin Virus führte zu verzögerter Replikation und dadurch Attenuierung (Hall et al., 1999). Attenuierende Mutationen wurden auch in den Proteinen NS3 (Butrapet et al., 2000) und NS5 (Xie et al., 1998) beschrieben.

Nichtkodierende Genomabschnitte: Attenuierung des FSME Virus durch Deletionen in der 3'-terminalen nichtkodierenden Region wurde beschrieben (Mandl et al., 1998). Bei Dengue Viren wurden experimentelle Impfstoffe mit Deletionen in sowohl 5'- als auch 3'-nichtkodierenden Regionen hergestellt (Lai et al., 1998). Es wird angenommen, dass die molekulare Basis der Attenuierung dieser Viren die Beeinträchtigung der viralen Replikation durch diese Mutationen ist.

Die Aufgabe der vorliegenden Erfindung liegt nun darin, weitere Möglichkeiten zur Attenuierung von Flaviviren zur Verfügung zu stellen, die zu Lebendimpfstoffen fuhren, die vor allem zur Vorbeugung von Erkrankungen, die auf Flaviviren zurückzuführen sind, geeignet sind.

Diese Aufgabe wird erfindungsgemäß gelöst durch einen attenuierten Flaviviren-Lebendimpfstoff, umfassend eine Flavivirus-Mutante, welche dadurch gekennzeichnet ist, dass die Flavivirus-Mutante eine Deletion im Kapsidprotein von zumindest mehr als 4 aufeinanderfolgende Aminosäuren aufweist, wobei der carboxy-terminale hydrophobe Bereich vor der Deletion nicht betroffen ist. Obgleich - wie oben erwähnt - eine Reihe von attenuierten Flaviviren bereits im Stand der Technik beschrieben worden ist, deren Attenuierung auf Mutationen beruht, wurden bislang in keinem Fall Mutationen, insbesondere Deletionen, im Kapsidprotein als Grundlage der Attenuierung von Flaviviren beschrieben. Es war auch völlig überraschend, dass Flaviviren, die die erfindungsgemäße Mutation gerade im Kapsidprotein aufweisen, zu einer verlässlichen Attenuierung von Flaviviren führen und als Flavivirus-Lebendvakzine eingesetzt werden können. Trotz der erfindungsgemäß vorgesehenen Mutation im Kapsidprotein kann nämlich eine Vermehrung des attenuierten Virus im Geimpften nach Verabreichung des erfindungsgemäßen Virus als Lebendvakzine stattfinden. Daraus resultiert eine Reihe von Vorteilen gegenüber Totimpfstoffen.

Deletionen im Kapsidprotein stellen ein völlig neuartiges Attenuierungsprinzip für Flaviviren dar. Bislang wurde das Kapsidprotein in der Fachwelt in dieser Hinsicht nie als relevantes Objekt angesehen. Umso überraschender war daher die Erkenntnis der vorliegenden Erfindung, dass die beschriebenen Deletionsmutanten tatsächlich erfolgreich zu attenuierten Flavivirus-Lebendimpfstoffen führen, die resistent gegen eine Revertierung zum virulenten Phänotyp sind und sich daher vorzüglich für die breite Anwendung am Menschen eignen.

Für die Herstellung konventioneller Totimpfstoffe ist es nötig, große Mengen infektiösen und virulenten Virus zu produzieren. Auch bei rekombinant hergestellten Totimpfstoffen müssen große Mengen von Antigen produziert und gereinigt werden. Bei einer Lebendvakzine sind die zu produzierenden Mengen wesentlich geringer, da die Vakzine im Körper des Geimpften selbst vermehrt wird, wodurch die Produktionskosten von Lebendvakzinen im allgemeinen wesentlich günstiger sind als jene von Totimpfstoffen. Außerdem wird kein virulentes, sondern apathogenes Virus produziert, weshalb kein Gesundheitsrisiko bei der Produktion besteht. Konventionelle Flavivirus-Totimpfstoffe werden durch Inaktivierung infektiöser Partikel durch Behandlung mit Formalin hergestellt, wodurch eine gewisse Veränderung der Antigenstruktur hervorgerufen wird. Im Geimpften wird eine hauptsächlich humorale Immunantwort gegen Strukturproteine, deren Antigenstruktur nicht exakt der nativen Form entspricht, und keine Immunantwort gegen Nichtstrukturproteine, deren Bedeutung aber für den Aufbau einer langanhaltenden Immunität und der Bildung zytotoxischer T-Zellen sehr groß ist; gebildet.

Im Gegensatz dazu kann mit der erfindungsgemäßen Vakzine eine humorale und zelluläre Immunantwort gegen völlig nativ vorliegende und in vivo produzierte Oberflächen- und Nichtstrukturproteine erzielt werden, wodurch entsprechend dem gegenwärtigen Stand des Wissens eine wesentlich länger anhaltende schützende Immunantwort als bei einer Totvakzine erzielt werden kann.

Der erfindungsgemäße attenuierte Flaviviren-Lebendimpfstoff, insbesondere gemäß den bevorzugten Ausführungsformen, weist aber noch weitere Vorteile gegenüber konventionellen Lebendimpfstoffen und gentechnisch hergestellten experimentellen Lebendimpfstoffen auf:

Die in Gebrauch stehenden Flavivirus-Lebendimpfstoffe wurden vielfach im Labor passagiert, wobei eine Vielzahl von Mutationen entstand, deren Bedeutung für die Biologie dieser Viren im Einzelnen nicht vollständig nachvollziehbar ist und deren jeweiliger Beitrag zur Attenuierung dieser Viren sowie das Wechselspiel der einzelnen attenuierenden Mutationen sind nicht vollständig bekannt (für JE siehe: Nitayaphan et al., 1990; für GF siehe: Post et al., 1992; für CSF siehe: Bjorklund et al., 1998). Einige Mutationen befinden sich auch in für die Immunantwort besonders wichtigen Antigenen wie dem Oberflächenprotein E. Dadurch liegen gewisse Antigendeterminanten in gegenüber dem Wildvirus veränderter Form vor. Die Komplexizität der genetischen Basis der Attenuierung dieser Viren erlaubt es nicht, die der Attenuierung zu Grunde liegenden Prinzipien direkt auf andere Flaviviren anzuwenden.

Im Gegensatz dazu werden bei der erfindungsgemäßen Vakzine nur definierte und allgemein anwendbare attenuierete Mutationen im Kapsidprotein eingeführt, wodurch es nicht notwendig ist, ein für die Immunantwort besonders wichtiges Protein (das Hüllprotein oder gewisse nichtstrukturelle Proteine, wie NS1 beim Genus Flavivirus) zu verändern.

Bevorzugte Ausführungsformen des erfindungsemäßen Lebendimpfstoffes weisen daher keine weiteren Mutationen auf, insbesondere nicht in den Hüllproteinen sowie in anderen Proteinen, die die Immunantwort betreffen.

Wie ebenfalls oben erwähnt worden ist, ist eine Reihe von gentechnisch hergestellten attenuierten Flaviviren beschrieben worden, in welchen die Attenuierung auf Punktmutationen beruht. Diese können relativ einfach genetisch revertieren. Auch die Reversion eines durch Punktmutation attenuierten Virus zu einem virulentem Phänotyp durch eine zweite Punktmutation wurde beschrieben (Mandl et al., 2000). Im Gegensatz dazu wird beim erfindungsgemäßen Lebendimpfstoff die Attenuierung durch eine Deletion erzielt, deren Reversion zum Wildtyp ausgeschlossen ist.

In weiteren beschriebenen Fällen beruht die Attenuierung auf Veränderungen in für die Immunantwort wichtigen Hüllproteinen oder in Genomabschnitten, die für die Replikation und Translation wichtig sind. Weder eine Veränderung der Antigenstruktur von Hüllproteinen noch eine wesentliche Beeinträchtigung der Replikation oder Translation sind wünschenswert, wenn eine möglichst natürliche und effiziente Immunantwort hervorgerufen werden soll. Diese Nachteile werden von der vorliegenden Erfindung, bei der nur eine innere Strukturkomponente nicht aber Hüllproteine, Nichtstrukturproteine oder regulatorische nichtkodierende Abschnitte verändert wurden, überwunden.

In einem weiteren Ansatz wurden Flavivirus-Vakzine durch Kombination verschiedener Viren (Chimären) hergestellt (Guirakhoo et al., 2400). Da Chimären Organismen darstellen, in denen Gene pathogener Viren auf eine in der Natur nicht vorkommende Weise neu miteinander kombiniert werden, beinhaltet die Freisetzung solcher Viren durch Impfung das Risiko einer Evolution dieser Chimären zu neuen Viren deren Eigenschaften nicht vorhergesagt werden können. Im Gegensatz dazu stellt die neue Vakzine keine Kombination verschiedener Virusgenome dar und es ist daher nicht möglich, dass eine Freisetzung durch Impfung die Entstehung einer bisher in der Natur nicht vorkommenden Virusspezies verursachen könnte.

Die Einführung der erfindungsgemäßen Deletionen in das Kapsidprotein von Flaviviren ist beispielsweise mit Hilfe rekombinanter Techniken für jeden Fachmann unter Heranziehung an sich bekannter Verfahren ohne großen Experimentieraufwand möglich. Der für das jeweilige Kapsidprotein kodierende Genabschnitt ist für alle Flaviviren, deren Genomsequenz bisher aufgeklärt wurde, bekannt und kann für neue Flavivirussequenzen durch Sequenzvergleich leicht ermittelt werden. Die Deletionen dürfen dabei selbstverständlich zu keiner Verschiebung des Leserahmens führen, so dass der carboxy-terminale hydrophobe Bereich von der Deletion betroffen würde. Es ist essentiell, dass dieser carboxy-terminale hydrophobe Bereich weitgehend erhalten wird und daher von der Deletion nicht betroffen ist. Durch die erwähnten Techniken ist es möglich, mutante, infektiöse Viren herzustellen, bei deren Vermehrung alle viralen Proteine außer dem Kapsidprotein in nativer Form entstehen. Die Replikation und Translation dieser Viren ist dann nicht oder nicht wesentlich eingeschränkt. Von diesen Viren können durch Vermehrung in Zellkulturen Präparationen hergestellt werden, die als Impfstoff verwendet werden können. Im Gegensatz zum unveränderten Wildtypvirus zeigen die erfindungsgemäßen Viren nach Inokulation in einem entsprechenden Wirtsorganismus einen attenuierten Phänotyp, d.h. sie rufen keine Erkrankung hervor. Sie induzieren aber die Bildung einer spezifischen Immunantwort. Ein mit dem erfindungsgemäßen Flaviviren-Lebendimpfstoff immunisierter Wirtsorganismus ist gegen eine nachfolgende Infektion mit dem virulenten Wildtyp geschützt, d.h. im Gegensatz zum ungeschützten Organismus kommt es zu keiner Erkrankung durch das Wildtyp-Virus.

Die erfindungsgemäße Deletion im Bereich des Kapsidproteins eignet sich dann besonders gut zur Herstellung einer als Lebendvakzine geeigneten Mutante, wenn eine Reihe von Charakteristika, mit Hilfe derer die Eigenschaften des Impfstoffs bei der Herstellung einer als Vakzine geeigneten Mutante verbessert werden können, beachtet werden. Die erfindungsgemäß vorzusehenden Deletionen müssen jedenfalls größer als 4 Aminosäuren zu sein, um geeignete attenuierte immunogene Viren herzustellen, da Viren mit Deletionen von bis zu 4 Aminosäuren noch einen dem Wildtyp-Virus ähnlichen virulenten Phänotyp zeigen. Darüber hinaus kann auch erst ab einer Deletion von dieser Größe eine Revertierung zum virulenten Virustyp ausgeschlossen werden.

Weiters dürfen die erfindungsgemäßen Deletionen nicht den carboxy-terminalen hydrophoben Bereich des Kapsidproteins betreffen. Es ist bekannt, dass diese Sequenz für die korrekte Bildung des im Genom nachfolgenden Hüllproteins notwendig ist und durch eine proteolytische Spaltung von reifen Kapsidprotein entfernt wird. Die Länge dieser Signalsequenz ist in den einzelnen Flavivirus-Typen unterschiedlich, kann aber leicht durch Erstellung von Hydrophilizitätsprofilen (vgl. Fig. 1) aufgefunden werden. Demgemäß darf die erfindungsgemäße Deletion diesen carboxy-terminalen Bereich nicht betreffen.

Der carboxy-terminale hydrophobe Bereich betrifft zumindest alle Aminosäuren in diesem Bereich, die einen Hydrophilie-Score gemäß Fig. 1 von -1 und weniger aufweisen. Besonders bevorzugt bleibt der C-terminale Bereich mit einer Hydrophilie unter 0 gemäß Fig. 1 unverändert.

Bevorzugte erfindungsgemäße Deletionen betreffen Bereiche von internen hydrophoben Domänen. Aus Fig. 1 ist ersichtlich, dass die Kapsid-Sequenzen aller Flaviviren neben der oben genannten hydrophoben Signalsequenz am Carboxy-Terminus noch Abschnitte mit überwiegend hydrophobem Charakter inmitten der sonst überwiegend hydrophilen Aminosäurekette enthalten. Deletionen, womit Bereiche dieser internen Domänen teilweise oder ganz entfernt werden, führen zu besonders geeigneten attenuierten Flaviviren-Lebendimpfstoffen. Als "interene hydrophobe Domäne" können zumindest alle Bereiche angesehen werden, die in Fig. 1 mit einem negativen Hydrophiliewert ausgewiesen sind.

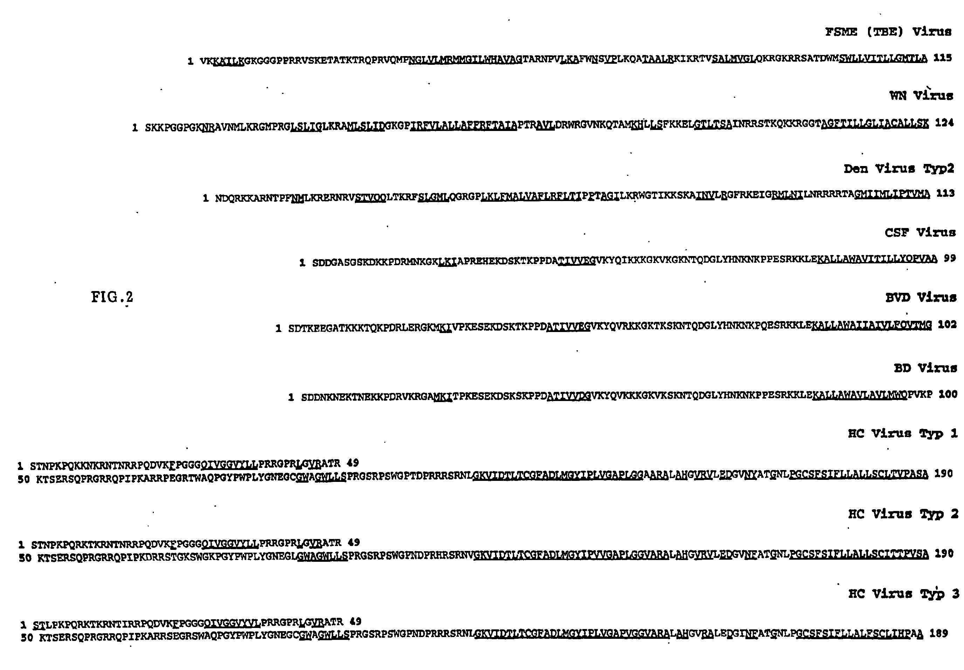

Besonders bevorzugte hydrophobe Bereiche, die in einer erfindungsgemäßen Vakzine zumindest teilweise deletiert werden können, sind in Fig. 2 für eine Reihe von Flaviviren gekennzeichnet. Diese Bereiche können durch Berechnung der Hydrophobizitäts-Profile der jeweiligen Aminosäuresequenzen bestimmt werden. Die in Fig. 2 unterstrichenen Bereiche wurden mit dem Algorithmus von Kyte und Doolittle (1982) mit einer Fenstergröße von 5 berechnet. Alternativ dazu können hydrophobe Bereiche auch mit Fenstergrößen zwischen 5 und 13 Aminosäureresten berechnet werden oder auch durch andere Algorithmen wie den von Hopp und Woods (1981), wobei üblicherweise Fenstergrößen zwischen 3 und 11 gewählt werden können.

Die Hydrophilizitäts-Blots gemäß Fig. 1 sind gemäß den Algorithmen von Kyte und Doolittle (1982) mit einer Fenstergröße von 9 Aminosäureresten bzw. Hopp und Woods (1981) mit einer Fenstergröße von 7 Aminosäureresten berechnet worden. Bevorzugte hydrophobe Bereiche sind ausgewählt aus Dengue 1: 46-67, Dengue 2: 46-66, Dengue 3: 46-67, Dengue 4: 45-65, Japanese Encephalitis: 46-62, West Nil: 46-62, Murray Valley Encephalitis: 46-62, Saint Louis Encephalitis: 45-61, Gelbfieber: 43-58, Langat: 42-54, Powassan: 40-52, FSME: 42-54 und HCV:119-145, insbesondere 125-144.

Eine besondere Ausführungsform der attenuierten Vakzine stellen Kapsiddeletionen dar, die so große Anteile einer internen hydrophoben Domäne entfernen, dass die resultierenden Mutanten Genome keine in Zellkultur passagierbaren Viren erzeugen. Diese Genome sind in der Lage zu replizieren und alle Proteine außer dem Kapsidprotein in nativer Form effizient zu translatieren und können eventuell auch nichtinfektiöse subvirale Partikel bilden. Solche Deletionsmutanten können durch die Einführung zusätzlicher Punktmutationen oder von Insertionen, die die Hydrophobizität des Kapsidproteins erhöhen, zu passagierbaren und als Lebendimpfstoff geeigneten Virusmutanten gemacht werden. Bei den Insertionen handelt es sich dabei um Sequenzen, die von der ursprünglich deletierten Sequenz unterschiedlich sind. Geeignete Zusatzmutationen können beispielsweise dadurch generiert und identifiziert werden, dass die Deletionsmutanten, die in einem Zellkultursystem nicht mehr replikationsfähig sind, in ein empfindlicheres Vermehrungssystem (beispielsweise Labormäuse für Viren des Genus Flaviviren, die natürlichen Wirte der Pestiviren Schwein, Schaf, Rind, oder Schimpansen im Fall des Hepatitis C Virus) als die Zellkultur es ist, eingebracht werden und durch Passagierung in diesem System entsprechende Mutationen generiert und selektioniert werden. Besonders bevorzugte empfindlichere Systeme sind dabei Tiere, insbesondere Mäuse, Ratten, Kaninchen, Hasen, Schweine, Schafe, Rinder oder Primaten, z.B. Schimpansen. Die resultierenden Virusmutanten stellen eine besonders sichere Vakzine dar, da etwaige Reversion der Zusatzmutation zu nicht oder stark eingeschränkt vermehrungsfähigem Virus führen würde. Eine Reversion der Deletion ist a priori unmöglich.

Bevorzugte attenuierte Flaviviren-Lebendimpfstoffe umfassen eine Flavivirus-Mutante, welche eine Deletion von 5 bis 70, vorzugsweise 6 bis 25, insbesondere 7 bis 20, aufeinanderfolgende Aminosäuren im Kapsidprotein aufweisen. Deletionen dieser bevorzugten Größen zeichnen sich durch jeweils qualitativ veränderte Eigenschaften in ihrem Attenuierungs-/Passagierungs-Verhalten aus. Wenn die Deletionen größer als 20 aufeinander folgende Aminosäuren sind, kann es zu einem Verlust der Passagierbarkeit bei der Herstellung des Lebendimpfstoffes in Wirtszellen kommen. Diese fehlende Passagierbarkeits-Eigenschaft ist abhängig von der Empfindlichkeit der Wirtszellen, kann aber durch bestimmte Zusatzmutationen für die betreffenden Wirtszellen wiederhergestellt werden. Die Wiederherstellung kann durch eine oder mehrere Mutationen erfolgen, mit denen die Hydrophobizität des Kapsidproteins erhöht wird. Diese weiteren Mutationen sind vorzugsweise ausgewählt aus Punktmutationen, mit denen die Hydrophobizität des Kapsidproteins erhöht wird oder indem Insertionen von Aminosäureabschnitten mit vorwiegend hydrophoben Eigenschaften in das Kapsidprotein-Gen eingeführt werden. Besonders bewährt haben sich Duplikationen von hydrophoben Bereichen des Kapsidproteins. Besonders bevorzugte Punktmutationen umfassen den Austausch von geladenen bzw. hydrophilen Aminosäuren (wie z.B. Asparaginsäure, Glutaminsäure, Lysin, Arginin, Histidin, Tryptophen, Glutamin, Asparagin, Tyrosin, Serin, etc.) zu weniger polaren Aminosäuren bzw. unpolaren Aminosäuren (wie z.B.Isoleucin, Leucin, Phenylalanin, Methionin, Alanin, Valin etc.). Besonders bevorzugt werden derartige Zusatzmutationen durch die Einbringung in das empfindlichere System automatisch generiert. Selektion dieser Zusatzmutationen erfolgt ebenfalls automatisch durch Wiederherstellung der Passagierbarkeit selbst.

Oft reicht auch schon eine Mutation, die nur zu einem graduellen Unterschied in der Polarität der Aminosäure resultiert, um die Passagierbarkeit wiederherzustellen (beispielsweise der Austausch von Prolin gegen Leucin oder von Valin gegen Phenylalanin).

Bevorzugterweise können die Deletionen bis zu 15 Aminosäuren an den Amino-terminus und/oder bis unmittelbar vor Beginn der carboxy-terminalen Signalsequenz reichen. Obgleich bislang angenommen worden ist, dass die ersten 20 Aminosäuren des Flavivirus-Kapsidproteins (bzw. der für diese Aminosäure kodierende Genomabschnitt) für die Replikation absolut notwendig sind (Khromykh und Westaway, 1997), konnten im Rahmen der vorliegenden Erfindung auch replikationsfähige und vermehrungsfähige Viren mit Deletionen, die in diesen Bereich hineinragen, erfolgreich hergestellt werden.

Die vorliegende Erfindung ist für alle Vertreter der Flaviviren anwendbar. Der Begriff "Flaviviren" bezieht sich daher im Rahmen dieser Anmeldung auf alle Vertreter der Familie Flaviviridae, außer es wird ausdrücklich darauf hingewiesen, dass nur Vertreter des Genus Flavivirus gemeint sind. Besonders bevorzugte Vertreter von Flaviviren, mit denen die vorliegende Erfindung realisiert wird, sind ausgewählt aus der Gruppe bestehend aus Gelbfieber-Virus, Virus der japanischen Enzephalitis, den vier Serotypen der Dengue-Viren, Frühsommermeningoenzephalitis-Virus, West-Nil-Virus, Murray Valley Enzephalitis Virus, Saint Louis Enzephalitis Virus, Powassan Virus, klassisches Schweinefieber-Virus, bovines virales Diarrhoe-Virus, border disease-Virus, Hepatitis C-Virus und Hepatitis G-Virus. Diese Vertreter sind wegen ihrer bekannten Pathogenität für Mensch und Tier besonders geeignet für die vorliegende Erfindung, da für diese Vertreter der Bedarf für einen geeigneten attenuierten Lebendimpfstoffbesonders groß ist.

Für den erfindungsgemäßen Lebendimpfstoff ist nur eine geringe Menge an Virus für eine effiziente Immunisierung notwendig, so dass der erfindungsgemäße Lebendimpfstoff pro Verabreichung mit 10¹ bis 10⁷, vorzugsweise mit 10² bis 10⁶, insbesondere 10³ bis 10⁵, infektiösen Einheiten an Flaviviren das Auskommen findet. Der Lebendimpfstoff kann bevorzugterweise als Einmal-Dosis mit dieser Menge an infektiösen Einheiten verabreicht werden.

Bevorzugterweise enthält der erfindungsgemäße Lebendimpfstoff weitere wirksame Substanzen oder Hilfssubstanzen. Besonders bevorzugt ist dabei der Zusatz von Antibiotika wie Neomycin oder Kanamycin, Konservierungsmittel wie Thiomersal, und Stabilisatoren wie Humanalbumin, Lactose - Sorbitol, Sorbitol - Gelatine, Polygeline oder Salze wie MgCl₂ oder Mg SO₄. Allgemein können als Zusatzstoffe bevorzugterweise Aminosäuren, Polysaccharide und (Puffer-)Salze verwendet werden.

Bei der Herstellung des erfindungsgemäßen Lebendimpfstoffes empfiehlt sich - wenn die Verabreichung an Menschen vorgesehen ist - nicht transformierte Wirtszellen zu verwenden, da damit sowohl das Risiko an Eigenschaftsveränderungen (z.B. leichte Einführung von neuen, unerwünschten Mutationen) als auch das Kontaminationsrisiko mit Bestandteilen dieser Zellen hintangehalten wird.

Gemäß einem weiteren Aspekt der vorliegenden Erfindung kann der Flavivirus-Impfstoff auch als Nukleinsäure-Vakzine bereitgestellt werden, wobei die Nukleinsäure für ein erfindungsgemäß deletiertes Kapsidprotein kodiert. Bevorzugterweise enthält ein derartiger Nukleinsäure-Flavivirus-Impfstoff zusätzlich zur Nukleinsäure Aminoglycosid-Antibiotika, wie Neomycin oder Kanamycin, wie von der FDA für Plasmidvakzinen empfohlen. Für die Vakzinierierung mit "nackten" Nukleinsäuren ist im Stand der Technik eine ganze Reihe verschiedenster Strategien beschrieben worden (s. z.B. WO 90/11092, WO 94/29469, WO 97/47197, mit Bezugnahme hierin eingeschlossen; Liposomen-vermittelte Nukleinsäure-Transfer, vorzugsweise Nukleinsäure-Transfer mit (vorzugsweise biodegradierbaren) Mikrosphären, ...).

Die vorliegende Erfindung betrifft weiters auch ein Verfahren zur Herstellung des erfindungsgemäßen Lebendimpfstoffes, welches durch die folgenden Schritte gekennzeichnet ist:
· Bereitstellen eines Flavivirus oder einer Flavivirus-Nukleinsäure, wobei das Flavivirus bzw. die Flavivirus-Nukleinsäure eine erfindungsgemäße Deletion im Kapsidprotein aufweist,
· Vermehren des Flavivirus oder der Flavivirus-Nukleinsäure in geeigneten Wirtszellen,
· Gewinnen der durch die Wirtszellen vermehrten Viruspartikel und
· Aufarbeiten der Viruspartikel zu einem Lebendimpfstoff.

Bevorzugte Wirtszellen sind dabei ausgewählt aus embryonierten Hühnereiern, primären embryonalen Hühnerzellen, humanen diploiden Zell-Linien (z.B. WI-38, MRC-5), Verozellen, primären Hamsternierenzellen, primären Hundenierenzellen oder diploiden fötalen Rhesuslungenzellen.

Schließlich betrifft die vorliegende Erfindung auch die Verwendung einer Flavivirus-Nukleinsäure, welche eine erfindungsgemäße Deletion im Kapsidprotein aufweist, zur Herstellung eines Impfstoffes zur Prävention von Flavivirus-Infektionen.

Die Erfindung wird anhand der nachfolgenden Beispiele sowie der Zeichnungsfiguren, auf die sie jedoch nicht eingeschränkt sein soll, näher erläutert.

Es zeigen: Fig. 1A-C Hydrophilizitätsprofile der Kapsidproteine von je 3 Vertretern der 3 Genera der Familie Flaviviridae. Negative Werte zeigen Regionen mit überwiegend hydrophobem Charakter. Die carboxy-terminale hydrophobe Region ist die Signalsequenz für das im Genom folgende Hüllprotein. Für jedes Protein wurde die Hydrophilizität beispielhaft je einmal nach dem Algorithmus von Kyte und Doolittle (1982) mit einer Fenstergröße von 9 Aminosäureresten (oben) und nach dem Algorithmus von Hopp und Woods (1981) mit einer Fenstergröße von 7 Aminosäureresten (unten) berechnet und dargestellt.

Fig. 2. Sequenz-Anordnung der Kapsidproteine von je 3 Vertretern der 3 Genera der Familie Flaviviridae. Sequenzabschnitte mit überwiegend hydrophoben Charakter (bestimmt nach Kyte und Doolittle mit einer Fenstergröße von 5) sind unterstrichen. Dabei stellt der jeweils am weitesten carboxyterminal gelegene Abschnitt die Signalsequenz für das nachfolgende Hüllprotein dar. In diesem Abschnitt dürfen keine Deletionen erfolgen. Die anderen (internen) hydrophoben Abschnitte stellen bevorzugte Bereiche für attenuierende Deletionen dar.

### Beispiele :

### Beispiel 1: Verwendung einer Kapsiddeletionsmutante des FSME Virus als Lebendvakzine

Pathogenität und Immunogenität können im Fall des FSME Virus im adulten Mausmodell untersucht werden. Als Lebendvakzine wurde eine Mutante (CD28-43) des FSME Virus verwendet, die eine 16 Aminosäuren lange Deletion im Bereich des Kapsidproteins aufweist. Diese und in folgenden Beispielen besprochene andere Mutanten sind in Tabelle 1 zusammengefasst. Die Mutante CD28-43 zeigt eine dem Wildtypvirus vergleichbare Genexpression und kann in BHK-21 Zellen beliebig passagiert werden. Die Mutante wurde durch Einführung der entsprechenden Mutation in den infektiösen cDNA Klon des FSME Virus hergestellt. Von diesem mutierten cDNA Klon wurde RNA in vitro transkribiert und diese RNA in BHK-21 Zellen elektroporiert. Nach 3 Tagen wurde der Zellkulturüberstand geerntet und das Virus zweimal in Babymausgehirnen passagiert um eine hochtitrige Virussuspension zu erhalten. Diese wurde verwendet um die Eignung der Mutante als Lebendvakzine im adulten Mausmodell zu untersuchen. Dazu wurden je 10 5-Wochen-alte Mäuse mit 10,000 p.f.u. der Mutante oder des virulenten Wildtypvirus subkutan inokuliert und die Überlebensraten wurden über einen Zeitraum von 4 Wochen beobachtet. Wie in Tabelle 2 ersichtlich starben im Fall des virulenten Wildtypvirus 9 der 10 Mäuse mit den typischen klinischen Zeichen der Enzephalitis. Im Gegensatz dazu starb keine der mit der Kapsiddeletionsmutante inokulierten Mäuse. Auch bei Inokulation mit einer 100-fach höheren Dosis mit der Mutante erkrankte keine der 10 Mäuse. Dieses Ergebnis bedeutet, dass die Kapsiddeletionsmutante apathogen war. Eine Untersuchung von Serumproben aller mit der Mutante inokulierten Mäuse zeigte, dass alle eine spezifsiche Immunantwort gegen das FSME Virus ausgebildet hatten. Durch Inkulation mit einer hohen Dosis virulenten Virus wurde untersucht, ob diese Immunantwort gegen die Infektion mit dem Wildvirus zu schützen vermochte. Alle Mäuse überlebten diesen "Challenge" ohne Erkrankungszeichen. Dieses Ergebnis bedeutet, dass die Kapsiddeletionsmutante eine schützende Immunität hervorgerufen hatte und deshalb als Vakzine gegen das Wildvirus verwendet werden kann.

### Beispiel 2: Gewinnung einer passagierbaren Mutante mit einer Deletion, die in den Bereich der aminoterminalen 20 Aminosäuren reicht.

Mit Hilfe des infektiösen cDNA Klons wurde eine Deletion in das Kapsidprotein des FSME Virus eingeführt, die von Aminosäure 16 bis zur AS 25 reichte (Mutante CD16-25; siehe Tabelle 1). Mutante CD16-25 war in Zellkultur beliebig passagierbar. Dieses Ergebnis zeigt, dass im Gegensatz zum bisherigen Stand des Wissens Deletionen, die in den Bereich der 20 aminoterminalen Aminosäuren bis einschließlich der Aminosäure 16 hineinreichen, die Replikation von Flaviviren nicht zerstören.

### Beispiel 3: Virulente Kapsiddeletionsmutanten

Die Kapsiddeletionsmutanten CD28 und CD28-31 tragen Deletionen von 1 bzw. 4 Aminosäuren Länge (Tabelle 1). Die deletierten Aminosäuren sind bei diesen Mutanten nicht Teil einer internen hydrophoben Domäne (Fig. 1). Inokulation dieser Mutanten in erwachsene Mäuse zeigte, dass sie einen dem Wildtypvirus entsprechenden virulenten Phänotyp besitzen und 80% bzw. 100% der Mäuse töteten (Tabelle 2). Dieses Ergebnis zeigt, dass Deletionen bis zu einer Größe von 4 Aminosäuren in nicht hydrophoben Bereichen des Kapsidproteins zur Herstellung einer attenuierten Vakzine nicht geeignet sind.

### Beispiel 4: Attenuierte Kapsiddeletionsmutanten

Die Kapsiddeletionsmutanten CD28-35, CD28-39 und die in Beispiel 1 beschriebene Mutante CD28-43 tragen Deletionen von 8, 12 und 16 Aminosäuren Länge (Tabelle 1). Diese Deletionen entfernen Teile einer internen hydrophoben Domäne (Fig. 1). Inokulation dieser Mutanten in erwachsene Mäuse zeigte, dass sie einen attenuierten Phänotyp besitzen, d.h. keine oder im Fall der Mutante CD28-35 nur 10% der Mäuse töteten (Tabelle 2). Dieses Ergebnis zeigt, dass Deletionen, die Teile einer internen hydrophoben Domäne entfernen zur Attenuierung führen. Das Ausmaß der Attenuierung hängt dabei auch von der Größe der Deletion ab und kann durch Vergrößerung vermehrt werden. Alle mit diesen attenuierten Mutanten immunisierten Mäuse waren gegen Infektion mit dem virulenten Wildtyp Virus vollkommen geschützt. Dieses Ergebnis bestätigt das Ergebnis von Beispiel 1, dass Kapsiddeletionsmutanten als Lebendvakzinen verwendet werden können.

### Beispiel 5: Identifizierung von Zusatzmutationen

Die Kapsiddeletionsmutanten CD28-48, CD28-54 und CD28-89 tragen Deletionen von 21, 27 und 62 Aminosäuren Länge (Tabelle 1). Diese Deletionen entfernen eine interne hydrophobe Domäne zur Gänze bzw. entfernen im Falle der größten Deletion alle internen hydrophoben Abschnitte des Kapsidproteins (Fig. 1). Diese Mutanten zeigen eine ungestörte Genexpression, sind aber in BHK-21 Zellen nicht passagierbar. Überstände von mit diesen Mutanten transfizierten BHK-21 Zellkulturen wurden nach 3 Tagen geerntet und in je 10 Babymäuse (1 Tag alt) intracranial inokuliert. Dies stellt das empfindlichste der bekannten Vermehtungssysteme für das FSME Virus dar. Nach 5 bis 10 Tagen starben im Fall der Mutante CD28-48 alle, im Fall der Mutante CD28-54 7 der 10 Babymäuse unter deutlichen klinischen Zeichen der Enzephalitis. Bei den mit der Mutante CD28-89 inokulierten Mäusen verstarb keine. In den Gehirnen aller verstorbenen Mäuse konnte durch PCR das FSME Virus nachgewiesen werden, während kein Virus in den überlebenden Mäusen gefunden wurde. Virus aus den Gehirnen verstorbener Mäuse konnte in BHK-21 Zellkulturen beliebig passagiert werden. Dieses Ergebnis zeigt, dass bei Mutanten, die durch die Entfernung einer hydrophoben Domäne ihre Passagierbarkeit in Zellkultur verloren hatten, Revertanten selektioniert werden können, die wieder in Zellkultur passagierbar sind.

Um die genetische Grundlage dieser Veränderungen zu ermitteln wurden der für das Kapsidprotein kodierende Genomabschnitt für eine Reihe solcher Revertanten sequenziert. Es zeigte sich, dass in jedem Fall eine weitere Mutation im Protein C zusätzlich zu der ursprünglichen eingeführten Deletion vorhanden war. Dabei handelte es sich um Punktmutationen oder eine Sequenzduplikation. Diese zusätzlichen Mutationen sind in Tabelle 3 zusammengefasst. Ein gemeinsames Merkmal aller dieser Mutationen ist; dass sie die Hydrophobizität des Kapsidproteins erhöhen. Das Ergebnis zeigt, dass Mutationen, die die Hydrophobizität des Kapsidproteins erhöhen den Effekt von Deletionen interner hydrophober Sequenzen in Bezug auf die Passagierbarkeit von Flaviviren in Zellkulturen revertieren können.

### Beispiel 6: Kapsiddeletionsmutanten mit Zusatzmutationen als Lebendvakzine

Es wurden mit Hilfe des infektiösen Klons zwei Mutanten hergestellt, die eine 21 Aminosäuren lange Deletion der internen hydrophoben Domäne gemeinsam mit je einer Zusatzmutation (ausgewählt aus den in Tabelle 3 angegebenen Mutationen) im Kapsidprotein enthielten. Diese beiden Mutanten, CD28-43/L70 und CD28-43/Du8 waren in Gegensatz zu der entsprechenden Deletionsmutante ohne Zusatzmutation (CD28-43) in BHK-21 Zellen beliebig passagierbar (Tabelle 1). Dieses Ergebnis bestätigt die unter Beispiel 5 gemachten Aussagen. Inokulation dieser Mutanten in erwachsene Mäuse ergab, dass beide apathogen waren und eine schützende Immunität hervorriefen (Tabelle 2). Dieses Ergebnis zeigt, dass Mutanten mit Deletionen und Zusatzmutationen nach den oben beschriebenen Gesichtspunkten als Lebendvakzine geeignet sind.

**Tabelle 1 Kapsid-Deletionsmutanten des FSME Virus**

| Bezeichnung | Deletion^{a} | Zusatzmutation | Genexpression^{b} | Passagierbar keit^{c} |
|---|---|---|---|---|
| CD28 | Q28 | | + | + |
| CD28-31 | Q28-V31 | | + | + |
| CD28-35 | Q28-N35 | | + | + |
| CD28-39 | Q28-L39 | | + | + |
| CD28-43 | Q28-M43 | | + | + |
| CD28-46 | Q28-L46 | | + | + |
| CD28-48 | Q28-H48 | | + | - |
| CD28-54 | Q28-A54 | | + | - |
| CD28-89 CD16-25 | Q28-L89 R16-K25 | | + | - |
| | | | + | + |
| CD28-48/L70 | Q28-H48 | Q70->L | + | + |
| CD28-48/Du8 | Q28-H48 | 178-L85 Dupl^{d} | + | + |

| | | | | |
|---|---|---|---|---|
| ^{a}Angegeben sind die erste und letzte deletierte Aminosäure. Die Zahlen bezeichnen die Position der AS gezählt vom Aminoterminus des Kapsidproteins. | | | | |
| ^{b}Bestimmt durch Immunfluoreszenzfärbung von BHK-21 Zellen mit FSME spezifischen Antiseren. + = Immunfluoreszenzfärbung in vergleichbarer Intensität wie Wildtyp-Virus; - keine Färbung. | | | | |
| ^{c}Bestimmt durch mehrmalige Übertragung von Zellkulturüberständen auf BHK-21 Zellen: + passagierbar; - nicht passagierbar. | | | | |
| ^{d}Duplikation der 8 Aminosäuren von Position 78 bis 85 | | | | |

**Tabelle 2 Immunisierung erwachsener Mäuse**

| Inokulum | Menge^{a} | überlebend^{b} | schützend immunisiert^{c} |
|---|---|---|---|
| Wildtyp | 10⁴ pfu | 1/10 | n.a. |
| CD28 | 10⁴ pfu | 0/10 | n.a. |
| CD28-31 | 10⁴ pfu | 2/10 | n.a. |
| CD28-35 | 10⁴ pfu | 9/10 | 9/10 |
| CD28-39 | 10⁴ pfu | 10/10 | 10/10 |
| CD28-43 | 10⁴ pfu | 10/10 | 10/10 |
| CD28-43 | 10⁶ pfu | 10/10 | 10/10 |
| CD28-48/L70 | 10⁴ pfu | 10/10 | 10/10 |
| CD28-48/Du8 | 10⁴ pfu | 10/10 | 10/10 |

| | | | |
|---|---|---|---|
| ^{a} Menge des subkutan applizierten Inokulums angegeben in plaque forming units (pfu) pro Maus. | | | |
| ^{b} Zahl der Überlebenden Mäuse/Gesamtzahl der Mäuse | | | |
| ^{c} Zahl der immunisierten Mäuse/Gesamtzahl der Mäuse. Die Immunisierung wurde mit Hilfe des Nachweis von FSME-spezifischen Antikörpern im Serum bestimmt. Durch nachfolgende Infektion mit virulentem Wildvirus wurde bestätigt, dass die Immunisierung vollständig vor der Erkrankung schützte. n.a.=nicht anwendbar | | | |

**Tabelle 3 Kompensierende Mutationen in Kapsiddeletionsmutanten**

| RNA^{a} | Protein^{b} |
|---|---|
| A299->U | D56->I |
| C302->U | P57->L |
| G328->U | V66->F |
| A341->U | Q70->L |
| C347->U | T72->I |
| A368->U | K79->I |
| C374->U | T81->M |
| A401->U | Q90->L |
| 364-387 Dupl^{c} | 178-L85 Dupl |
| 307-363 Dupl | L59-K77 Dupl |

| | |
|---|---|
| ^{a} Nukleotidpositionen im Genom des FSME Virus (GenBank Nr. U27495) | |
| ^{b} Aminosäureposition in Protein C | |
| ^{c} Duplikation des angegebenen Abschnitts | |

### Literaturliste:

1. Bjorklund, H.V., T. Stadejek, S. Vilcek, und S. Belak. 1998. Molecular characterization of the 3' noncoding region of classical swine fever virus vaccine strains. Virus Genes 16:307-312.
2. Butrapet, S., C. Y. Huang, D. J. Pierro et al. 2000. Attenuation markers of a candidate dengue type 2 vaccine virus, strain 16681 (PDK-53), are defined by mutations in the 5' noncoding region and nonstructural proteins 1 and 3. J. Virol. 74:3011-3019.
3. Guirakhoo, F., R. Weltzin, T.J. Chambers et al. 2000. Recombinant chimeric yellow feverdengue type 2 virus is immunogenic and protective in nonhuman primates. J. Virol. 74:5477-5485.
4. Hall R.A., A.A. Khromykh, J.M. Mackenzie, et al. 1999. Loss of dimerisation ofthe nonstructural protein NS1 of Kunjin virus delays viral replication and reduces virulence in mice, but still allows secretion of NS1. Virology 264:66-75.
5. Hope, R.G., und J. McLauchlan. 2000. Sequence motifs required for lipid droplet association and protein stability are unique to the hepatitits C virus core protein. J. Gen. Virol. 81:1913-1925.
6. Hopp, T.P., und K.R. Woods. 1981. Prediction of protein antigenic determinants from amino acid sequences: PNAS 78:3824-3828.
7. Khromykh, A.A., und E.G. Westaway. 1996. RNA binding properties of core protein ofthe flavivirus Kunjin. Arch. Virol. 141:685-699.
8. Khromykh, A.A., und E.G. Westaway. 1997. Subgenomic replicons of the flavivirus Kunjin: construction and applications. J. Virol. 71:1497-1505.
9. Kyte, J., und R.F. Doolittle. 1982. A simple method for displaying the hydropathic character of a protein. J. Mol. Biol. 157:105-132.
10. Lai, C.J., M. Bray, R. Men, A. Cahour et al. 1998. Evaluation ofmolecular strategies to develop a live dengue vaccine. Clin Diagn. Virol. 10:173-179.
11. Mandl, C.W., Allison, S.L., Holzmann, H. et al. 2000. Attenuation of tick-borne encephalitis virus by structure-based site-specific mutagenesis of a putative flavivirus receptor binding site. J. Virol. 74:9601-9609.
12. Mandl, C.W., H. Holzmann, T. Meixner, S. Rauscher, P. F. Stadler, S. L. Allison, und F. X. Heinz 1998. Spontaneous and engineered deletions in the 3' noncoding region of tickbome encephalitis virus: Construction of highly attenuated mutants of a flavivirus. J. Virol. 72: 2132-2140.
13. Markoff, L., B. Falgout, und A. Chang. 1997. A conserved internal hydrophobic domain mediates the stable membrane integration of the dengue virus capsid protein. Virology 233:105-117.
14. McMinn, P. C. 1997. The molecular basis of virulence of the encephalitogenic flaviviruses. J. Gen. Virol. 78:2711-2722.
15. Meyers, G., A. Saalmuller, und M. Buttner. 1999. Mutations abrogating the Rnase activity in glycoprotein E(rns) of the pestivirus classical swine fever virus lead to virus attenuation. J. Virol. 73:10224-10235.
16. Nitayaphan, S., J. A. Grant, G.-J. J. Chang, und D. W. Trent. 1990. Nucleotide sequence of the virulent SA-14 strain of Japanese encephalitis virus and its attenuated derivative, SA-14-14-2. Virology 177:541-552.
17. Post, P. R., C. N. D. Santos, R. Carvalho, A. C. R. Cruz, C. M. Rice, und R. Galler. 1992. Heterogeneity in envelope protein sequences and N-linked glycosylation among yellow fever virus vaccine strains. Virology 188:160-167.
18. Xie, H., K.D. Ryman, G.A. Campbell, und A.D. Barrett. 1998. Mutation in NS5 protein attenuates mouse neurovirulence of yellow fever 17D vaccine virus. J. Gen. Virol. 79:1895-1899.

## Patentansprüche

1. Attenuierter Flaviviren-Lebendimpfstoff, umfassend eine Flavivirus-Mutante, **dadurch gekennzeichnet, dass** die Flavivirus-Mutante eine Deletion im Kapsidprotein von zumindest mehr als 4 aufeinanderfolgenden Aminosäuren aufweist, wobei der carboxy-terminale hydrophobe Bereich von der Deletion nicht betroffen ist.

2. Lebendimpfstoff nach Anspruch 1, **dadurch gekennzeichnet, dass** die Deletion eine interne hydrophobe Domäne des Kapsidproteins betrifft.

3. Lebendimpfstoff nach Anspruch 1 oder 2, **dadurch gekennzeichnet, dass** 5 bis 70, vorzugsweise 6 bis 25, insbesondere 7 bis 20, aufeinanderfolgende Aminosäuren im Kapsidprotein deletiert sind.

4. Lebendimpfstoff nach einem der Ansprüche 1 bis 3, **dadurch gekennzeichnet, dass** die Deletion größer als 20 aufeinanderfolgende Aminosäuren ist und das Kapsidprotein zusätzlich ein oder mehrere weitere Mutationen aufweist, mit denen die Hydrophobizität des Kapsidproteins erhöht wird, wobei die weiteren Mutationen vorzugsweise ausgewählt sind aus
• Punktmutationen, mit denen die Hydrophobizität des Kapsidproteins erhöht wird, oder
• Insertionen von Aminosäureabschnitten mit vorwiegend hydrophoben Eigenschaften, insbesondere Duplikationen von hydrophoben Bereichen des Kapsidproteins.

5. Lebendimpfstoff nach einem der Ansprüche 1 bis 4, **dadurch gekennzeichnet, dass** das Flavivirus ausgewählt ist aus der Gruppe bestehend aus Gelbfieber-Virus (GFV), Virus der japanischen Enzephalitis (JEV), den vier Serotypen der Dengue-Viren (DV), Frühsommermeningoenzephalitis-Virus (FSMEV), West-Nil-Virus (WNV), Murray Valley Enzephalitis- Virus (MVEV), Saint Louis Enzephalitis-Virus (SLEV), Powassan-Virus (PV), klassisches Schweinefieber-Virus (CSFV), bovines virales Diarrhoe-Virus (BDV), border disease Virus (BDV), Hepatitis C-Virus (HCV) und Hepatitis G-Virus (HGV).

6. Lebendimpfstoff nach einem der Ansprüche 1 bis 5, **dadurch gekennzeichnet, dass** er 10¹ bis 10⁷, vorzugsweise 10² bis 10⁶, insbesondere 10³ bis 10⁵, infektiöse Einheiten an Flaviviren enthält.

7. Lebendimpfstoff nach einem der Ansprüche 1 bis 6, **dadurch gekennzeichnet, dass** er weiters Antibiotika, Konservierungsmittel, Stabilisatoren, Pufferszubstanzen oder Mischungen davon umfasst.

8. Lebendimpfstoff nach Anspruch 7, **dadurch gekennzeichnet, dass** er Neomycin, Kanamycin, Thiomersal, Humanalbumin, Lactose-Sorbitol, Sorbitol-Gelatine, Polygeline, MgCl₂, MgSO₄, Aminosäuren, Polysaccharide, Puffersalze oder Mischungen davon umfasst.

9. Lebendimpfstoff nach einem der Ansprüche 1 bis 8, **dadurch gekennzeichnet, dass** er durch nicht-transformierte Wirtszellen hergestellt ist.

10. Flavivirus-Impfstoff, **dadurch gekennzeichnet, dass** er eine Nukleinsäure umfasst, die für ein deletiertes Kapsidprotein, wie in einem der Ansprüche 1 bis 5 definiert, kodiert.

11. Flavivirus-Impfstoff nach Anspruch 10, **dadurch gekennzeichnet, dass** er Aminoglykosid-Antibiotika, insbesondere Neomycin oder Kanamycin, Liposomen, Mikrosphären oder Mischungen davon umfasst.

12. Verfahren zur Herstellung eines Lebendimpfstoffes nach einem der Ansprüche 1 bis 9, **gekennzeichnet durch** die folgenden Schritte:
• Bereitstellen eines Flavivirus oder einer Flavivirus-Nukleinsäure, wobei das Flavivirus bzw. die Flavivirus-Nukleinsäure eine Deletion im Kapsidprotein von zumindest mehr als 4 aufeinanderfolgenden Aminosäuren, wie in einem der Ansprüche 1 bis 5 definiert, aufweist,
• Vermehren des Flavivirus oder der Flavivirus-Nukleinsäure in geeigneten Wirtszellen,
• Gewinnen der **durch** die Wirtszellen vermehrten Viruspartikel und
• Aufarbeiten der Viruspartikel zu einem Lebendimpfstoff.

13. Verfahren nach Anspruch 12, **dadurch gekennzeichnet, dass** die Wirtszelle ausgewählt ist aus embryonierten Hühnereiern, primären embryonalen Hühnerzellen, humanen diploiden Zell-linien, Verozellen, primären Hamsternierenzellen, primären Hundenierenzellen oder diploiden fötalen Rhesuslungenzellen.

14. Verwendung einer Flavivirus-Nukleinsäure, welche eine Deletion im Kapsidprotein von zumindest mehr als 4 aufeinanderfolgenden Aminosäuren, wie in einem der Ansprüche 1 bis 5 definiert, aufweist, zur Herstellung eines Impfstoffes zur Prävention von Flavivirus-Infektionen.

## Claims

1. An attenuated flavivirus live vaccine, comprising a flavivirus mutant, **characterised in that** the flavivirus mutant has a deletion in the capsid protein of at least more than 4 successive amino acids, wherein the carboxy-terminal hydrophobic region is not affected by the deletion.

2. A live vaccine according to claim 1, **characterised in that** the deletion concerns an internal hydrophobic domain of the capsid protein.

3. A live vaccine according to claim 1 or 2, **characterised in that** 5 to 70, preferably 6 to 25, in particular 7 to 20, successive amino acids in the capsid protein are deleted.

4. A live vaccine according to any one of claims 1 to 3, **characterised in that** the deletion is larger than 20 successive amino acids and the capsid protein additionally comprises one or more further mutations by which the hydrophobicity of the capsid protein is increased, the further mutations preferably being selected from
• point mutations by which the hydrophobicity of the capsid protein is increased, or
• insertions of amino acid sections having predominantly hydrophobic properties, in particular duplications of hydrophobic regions of the capsid protein.

5. A live vaccine according to any one of claims 1 to 4, **characterised in that** the flavivirus is selected from the group consisting of yellow fever virus (YFV), Japanese encephalitis virus (JEV), the four serotypes of the dengue viruses (DV), tick-borne encephalitis virus (TBE virus), West Nile virus (WNV), Murray Valley encephalitis virus (MVEV), Saint Louis encephalitis virus (SLEV), Powassan virus (PV), classical porcine fever virus (CPFV), bovine viral diarrhoea virus (BDV), border disease virus (BDV), hepatitis C virus (HCV) and hepatitis G virus (HGV).

6. A live vaccine according to any one of claims 1 to 5, **characterised in that** it comprises 10¹ to 10⁷, preferably 10² to 10⁶, in particular 10³ to 10⁵, infectious units of flaviviruses.

7. A live vaccine according to any one of claims 1 to 6, **characterised in that** it further comprises antibiotics, preservatives, stabilizers, buffer substances or mixtures thereof.

8. A live vaccine according to claim 7, **characterised in that** it comprises neomycin, kanamycin, thiomersal, human albumin, lactose-sorbit, sorbit-gelatine, polygeline, MgCl₂, MgSO₄, amino acids, polysaccharides, buffer salts or mixtures thereof.

9. A live vaccine according to any one of claims 1 to 8, **characterised in that** it is produced by non-transformed host cells.

10. A flavivirus vaccine, **characterised in that** it comprises a nucleic acid which codes for a deleted capsid protein as defined in any one of claims 1 to 5.

11. A flavivirus vaccine according to claim 10, **characterised in that** it comprises aminoglycoside antibiotics, in particular neomycin or kanamycin, liposomes, microspheres or mixtures thereof.

12. A method of producing a live vaccine according to any one of claims 1 to 9, **characterized by** the following steps:
· providing a flavivirus or a flavivirus nucleic acid, wherein the flavivirus or the flavivirus nucleic acid has a deletion in the capsid protein of at least more than 4 successive amino acids, as defined in any one of claims 1 to 5,
· propagating the flavivirus or the flavivirus nucleic acid in suitable host cells,
· recovering the virus particles propagated by the host cells, and
· working up the virus particles to a live vaccine.

13. A method according to claim 12, **characterised in that** the host cell is selected from chicken embryo cells, primary chicken embryo cells, human diploid cell lines, vero cells, primary hamster kidney cells, primary canine kidney cells or diploid fetal rhesus lung cells.

14. The use of a flavivirus nucleic acid which has a deletion in the capsid protein of at least more than 4 successive nucleic acids, as defined in any one of claims 1 to 5, for preparing a vaccine for the prevention of flavivirus infections.

## Revendications

1. Vaccin vivant atténué contre les flavovirus, comprenant un mutant du flavovirus, **caractérisé en ce que** le mutant du flavovirus présente dans la protéine de capside une délétion d'au moins plus de 4 acides aminés successifs, la zone hydrophobe carboxy-terminale n'étant pas concernée par la délétion.

2. Vaccin selon la revendication 1, **caractérisé en ce que** la délétion concerne un domaine hydrophobe interne de la protéine de capside.

3. Vaccin selon les revendications 1 ou 2, **caractérisé en ce que** 5 à 70, de préférence 6 à 25, en particulier 7 à 20 acides aminés successifs de la protéine de capside sont enlevés.

4. Vaccin vivant selon l'une des revendications 1 à 3, **caractérisé en ce que** la délétion concerne plus de 20 acides aminés successifs et **en ce que** la protéine de capside présente de plus une ou plusieurs autres mutations qui augmentent l'hydrophobicité de la protéine de capside, les autres mutations étant choisies de préférence parmi :
- des mutations ponctuelles augmentant l'hydrophobicité de la protéine de capside ou
- des insertions de tronçons d'acides aminés principalement à propriétés hydrophobes, en particulier de duplications de zones hydrophobes de la protéine de capside.

5. Vaccin vivant selon l'une des revendications 1 à 4, **caractérisé en ce que** le flavovirus est choisi dans le groupe constitué des virus de la fièvre jaune (GFV), des virus de l'encéphalite japonaise (JV), des quatre sérotypes des virus de la dengue (DV), du virus de la méningo-encéphalite du début de l'été (FSMEV), du virus du Nil occidental (WNV), du virus de l'encéphalite de la Vallée de Murray (MVEV), du virus de l'encéphalite de Saint-Louis (SLEV), du virus de Powassan (PV), du virus classique de la fièvre porcine (CSFV), du virus de la diarrhée bovine virale (BVDV), du virus de la maladie des frontières (BDV), du virus de l'hépatite C (HCV) et du virus de l'hépatite G (HGV).

6. Vaccin vivant selon une des revendications 1 à 5, **caractérisé en ce qu'**il contient 10¹ à 10⁷, de préférence 10² à 10⁶ et en particulier 10³ à 10⁵ unités infectieuses de flavovirus.

7. Vaccin vivant selon une des revendications 1 à 6, **caractérisé en ce qu'**il comprend en outre des antibiotiques, des agents de conservation, des agents de stabilisation, des substances-tampons ou leurs mélanges.

8. Vaccin vivant selon la revendication 7, **caractérisé en ce qu'**il comprend de la néomycine, de la canamycine, du thiomersal, de l'albumine humaine, du sorbitol lactose, de la gélatine sorbitol, de la polygéline, du MgCl₂, du MgSO₄, des acides aminés, des polysaccharides, des sels tampons ou leurs mélanges.

9. Vaccin vivant selon l'une des revendications 1 à 8, **caractérisé en ce qu'**il est préparé à base de cellules hôtes non transformées.

10. Vaccin contre le flavovirus, **caractérisé en ce qu'**il comprend un acide nucléique qui code pour une protéine de capside délétée selon l'une des revendications 1 à 5.

11. Vaccin contre le flavovirus selon la revendication 10, **caractérisé en ce qu'**il comprend des antibiotiques de la famille des aminoglycosides, en particulier la néomycine ou la canamycine, des liposomes, des microsphères ou leurs mélanges.

12. Procédé pour la préparation d'un vaccin vivant selon une des revendications 1 à 9, **caractérisé par** les étapes suivantes :
- préparation d'un flavovirus ou d'un acide nucléique de flavovirus, le flavovirus ou l'acide nucléique de flavovirus présentant dans la protéine de capside une délétion d'au moins plus de 4 acides aminés successifs selon une des revendications 1 à 5,
- multiplication du flavovirus ou des acides nucléiques de flavovirus dans des cellules hôtes appropriées,
- récolte des particules virales multipliées par les cellules hôtes et
- la transformation des particules virales en un vaccin vivant.

13. Procédé selon la revendication 12, **caractérisé en ce que** la cellule hôte est choisie parmi des oeufs de poule embryonnés, des cellules primaires embryonnaires de poule, des lignées de cellules humaines diploïdes, des vérocellules, des cellules rénales primaires de hamster, des cellules rénales primaires de chien ou des cellules pulmonaires diploïdes de foetus de rhésus.

14. Utilisation d'un acide nucléique de flavovirus qui présente dans la protéine de capside une délétion d'au moins plus de 4 acides aminés successifs selon une des revendications 1 à 5 pour la préparation d'un vaccin destiné à la prévention des infections par flavovirus.
